# DEMANDE DE BREVET EUROPEEN

(11) **EP 2 740 725 A1**
(43) Date de publication de la demande: **11.06.2014**
(21) Numéro de dépôt: 13195800.1
(22) Date de dépôt: 05.12.2013
(51) Int. Cl.: C07D 223/14, C07C 253/00

(54) **Nouveau procédé de synthèse du 3-(2-bromo-4,5-diméthoxyphényl)propanenitrile, et application à la synthèse de l'ivabradine et de ses sels d'addition à un acide pharmaceutiquement acceptable**

(30) Priorité: 06.12.2012 FR 1261714
(71) Demandeur: Les Laboratoires Servier, 92284 Suresnes Cedex (FR)
(72) Inventeur: Carranza, Maria del Pilar, 13670 Villarrubia de los Ojos (ES); Garcia Aranda, Maria Isabel, 45006 Toledo (ES); Gonzalez, José Lorenzo, 45007 Toledo (ES); Sanchez, Frédéric, 45111 Cobisa (ES)

(57) **Abrégé**

Procédé de synthèse du composé de formule (I) :

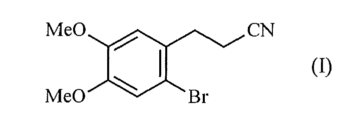

Application à la synthèse de l'ivabradine, de ses sels d'addition à un acide pharmaceutiquement acceptable et de ses hydrates.

## Description

La présente invention concerne un procédé de synthèse du (3-(2-bromo-4,5-diméthoxyphényl)propanenitrile de formule (I) : 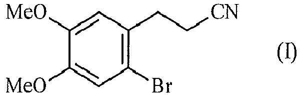 et son application à la synthèse de l'ivabradine et de ses sels d'addition à un acide pharmaceutiquement acceptable.

Le composé de formule (I) obtenu selon le procédé de l'invention est utile dans la synthèse de l'ivabradine de formule (II) : 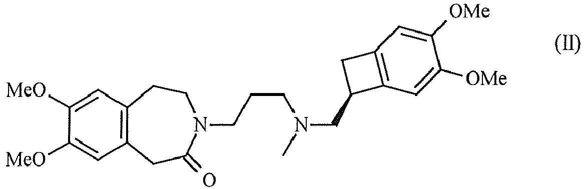 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
qui peut être transformée en un de ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique et en un de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrites dans le brevet européen EP 0534 859.

Ce brevet décrit la préparation de l'ivabradine à partir du 3,4-diméthoxybicyclo[4.2.0]octa-1,3,5-triène-7-carbonitrile de formule (III) : 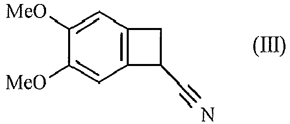 qui est transformé en composé de formule (IV) : 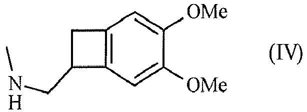 qui est dédoublé pour conduire au composé de formule (V) : 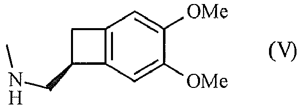 qui est mis en réaction avec le composé de formule (VI) : 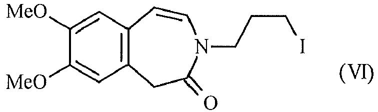 pour conduire au composé de formule (VII) : 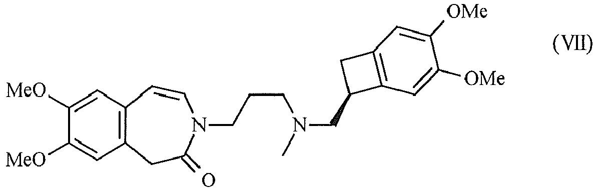 dont l'hydrogénation catalytique conduit à l'ivabradine, qui est alors transformée en son chlorhydrate.

La préparation du composé de formule (III) à partir du composé de formule (I) est décrite dans Tetrahedron 1973, 29, pp 73-76.

Ce même document décrit également une voie synthèse du composé de formule (I) à partir du 2-bromo-4,5-diméthoxybenzaldéhyde en trois étapes avec un rendement global de 65%.

Plus récemment, Zhao et al. ont décrit une synthèse du composé de formule (I) à partir du 3,4-diméthoxybenzaldéhyde en trois étapes avec 51% de rendement global (CN 101 407 474 A et J. Chem. Res. 2009, 7, pp 420-422)

Le composé de formule (I) est un intermédiaire clé dans la synthèse de l'ivabradine.

Compte-tenu de l'intérêt industriel de l'ivabradine et de ses sels, il était impératif de trouver un procédé performant permettant d'accéder au (3-(2-bromo-4,5-diméthoxyphényl)propanenitrile de formule (I) avec un excellent rendement.

La présente invention concerne un procédé de synthèse du composé de formule (I) : 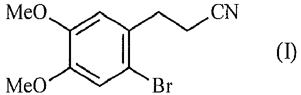 caractérisé en ce que le composé de formule (VIII) : 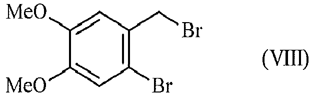 est soumis à l'action d'une base en présence d'acétonitrile dans un solvant organique pour conduire au composé de formule (I).

Parmi les bases pouvant être utilisées pour effectuer la transformation du composé de formule (VIII) en composé de formule (I), on peut citer à titre non limitatif le *n-*butyllithium, le diisopropylamidure de lithium, le bis(triméthylsilyl)amidure de potassium, le tert-butylate de potassium et l'hydroxyde de potassium.

La base préférentiellement utilisée pour effectuer la transformation du composé de formule (VIII) en composé de formule (I) est le *n*-butyllithium.

Parmi les solvants pouvant être utilisés pour effectuer la transformation du composé de formule (VIII) en composé de formule (I) on peut citer à titre non limitatif le tétrahydrofurane et l'acétonitrile.

Le solvant préférentiellement utilisé pour effectuer la transformation du composé de formule (VIII) en composé de formule (I) est le tétrahydrofurane.

La transformation du composé de formule (VIII) en composé de formule (I) est conduite à une température préférentiellement comprise entre -65°C et 25°C.

La présente invention concerne également un procédé de synthèse du composé de formule (I) à partir du composé de formule (VIII), caractérisé en ce que ledit composé de formule (VIII) est préparé à partir du composé de formule (IX) : 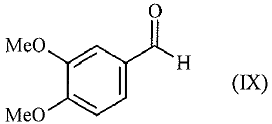 qui est transformé, par une réaction de réduction, en composé de formule (X) : 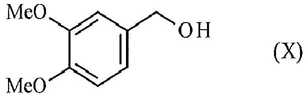 lequel est transformé, par une réaction de bromation, en composé de formule (VIII) : 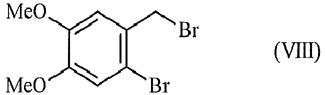 lequel est transformé en produit de formule (I) : 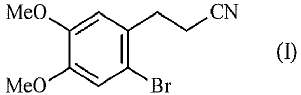 selon le procédé décrit plus haut.

La réaction de réduction du composé de formule (IX) peut être réalisée dans les conditions décrites dans la publication Org. Biomol. Chem. 2010, 8, 539-545.

La réaction de bromation du composé de formule (X) peut être réalisée dans les conditions décrites dans la publication Chem. Eur. J. 2010, 16, 9772-9776.

La présente invention concerne également un procédé de synthèse de l'ivabradine à partir du composé de formule (I) préparé selon le procédé de l'invention et transformé en composé de formule (III) en suivant l'enseignement de l'art antérieur (Tetrahedron 1973, 29, pp 73-76) par une réaction de cyclisation intramoléculaire en milieu basique ; ledit composé de formule (III) étant ensuite transformé en ivabradine selon le procédé décrit dans EP 0 534 859.

Les exemples suivants illustrent l'invention.

Le point de fusion a été mesuré avec BÜCHI Melting Point B-545 (Volt. 230VAC, Freq. 50/60 Hz, Power max. 220W).

### Liste des abréviations utilisées

P.F. : point de fusion

THF : tétrahydrofurane

### Préparation 1 : (3,4-diméthoxyphényl)méthanol D'après Org. Biomol. Chem. 2010, 8, 539-545

10 g (60,2 mmoles, 1éq.) de 3,4-diméthoxybenzaldéhyde sont dissous dans 300 mL de méthanol et la solution est refroidie à 0°C. 2,73 g (72,2 mmoles, 1,2 éq.) de NaBH₄ sont ajoutés par portions et le milieu réactionnel est agité 20 minutes puis hydrolysé par 10 mL d'une solution aqueuse d'HCl 1M jusqu'à ce que le milieu soit à pH neutre. Le solvant est éliminé sous pression réduite et le résidu est extrait par 3x50 mL de dichlorométhane. Les phases organiques sont rassemblées, séchées sur MgSO4, filtrées et concentrées sous pression réduite pour conduire à 9,88 g d'une huile claire.

Rendement = 98%

### Préparation 2 : 1-bromo-2-(bromométhyl)-4,5-diméthoxybenzène D'après Chem. Eur. J. 2010, 16, 9772-9776

A une solution de (3,4-diméthoxyphényl)méthanol (101,5 mmoles, 14,85 mL, 1éq.) dans 80 mL d'acide acétique glacial sont ajoutés sur 30 minutes à 0°C 6 mL de dibrome (116,8 mmoles, 1,15 éq.) dans 18 mL d'acide acétique glacial. Le milieu réactionnel est agité pendant 3h puis ramené à température ambiante. L'agitation est stoppée afin de permettre la précipitation totale du 1-bromo-2-(bromométhyl)-4,5-diméthoxybenzène au cours de la nuit. Le précipité est filtré, lavé au méthanol et recristallisé dans le méthanol pour conduire à 29,9 g d'une poudre jaune clair.

Rendement = 95%

### Exemple 1 : 3-(2-bromo-4,5-diméthoxyphényl)propanenitrile

A une solution de 0,3 mL d'acétonitrile (5,8 mmoles, 1,8 éq.) dans 15 mL de THF sont ajoutés à -60°C 1,77 mL de *n*-butyllithium (2 M dans du cyclohexane, 3,5 mmoles, 1,1 éq.). La solution est agitée 15 minutes à -60°C puis 1 g de 1-bromo-2-(bromométhyl)-4,5-diméthoxybenzène (3,2 mmoles) en solution dans 5 mL de THF est ajouté. Le milieu réactionnel est agité 1 heure puis hydrolysé par 10 mL d'eau et extrait 2 fois à l'acétate d'éthyle. Les phases organiques sont rassemblées et évaporées sous pression réduite. Le brut réactionnel est purifié par chromatographie sur gel de silice (éluant : méthylcyclohexane/acétate d'éthyle (70/30)). Après évaporation du solvant sous pression réduite, 505 mg d'une huile orange cristallisant sous forme de solide beige sont obtenus.

Rendement = 58%

P.F. = 74-81°C

### Exemple 2 : 3,4-diméthoxybicyclo[4.2.0]octa-1,3,5-triène-7-carbonitrile D'après Tetrahedron 1973, 29, pp 73-76

A une solution de NaNH₂, préparée à partir de 200 mL de NH₃ liquide et d' 1 g de Na (catalyseur FeCl₃), sont ajoutés par portions 5,4 g de 3-(2-bromo-4,5-diméthoxyphényl)propanenitrile et le mélange réactionnel est agité à température ambiante pendant 2 heures. Après évaporation de l'excès de NH₃, 2 g de NH₄Cl et 200 mL d'eau sont ajoutés par portions. Les cristaux gris formés sont collectés et recristallisés dans l'éthanol pour conduire à 2,38 g du produit attendu

Rendement = 74%

P.F. = 84-85°C

### Exemple 3 : 3,4-diméthoxy-N-méthylbicyclo[4.2.0]octa-1,3,5-trién-7-amine D'après EP 0 534 859

### Stade 1 : chlorhydrate de 3,4-diméthoxybicyclo[4.2.0]octa-1,3,5-trién-7-amine

On ajoute goutte à goutte et sous agitation à température ambiante, 312 mL d'une solution molaire de borane complexé avec du THF à une solution de 25 g de 3,4-diméthoxybicyclo[4.2.0]octa-1,3,5-triène-7-carbonitrile dans 250 mL de THF. On laisse en contact pendant 12 heures, puis on ajoute 200 mL d'éthanol et on agite 1 heure. On ajoute, goutte à goutte, 100 mL d'éther chlorhydrique 3,3N. On obtient 27,7 g du produit attendu.

Rendement = 90%

P.F. = 205°C

### Stade 2 : (3,4-diméthoxybicyclo[4.2.0]octa-1,3,5-trién-7-yl)carbamate d'éthyle

On coule 1,5 mL de chloroformiate d'éthyle sur une suspension de 3,4 g du composé obtenu au stade 1 de 4,5 mL de triéthylamine et de 50 mL de dichlorométhane. On laisse une nuit sous agitation à température ambiante puis on lave à l'eau et à l'acide chlorhydrique 1N. On sèche et on évapore à sec le solvant. On obtient 3,2 g d'une huile correspondant au produit attendu.

Rendement = 80%

### Stade 3 : 3,4-diméthoxy-N-méthylbicyclo[4.2.0]octa-1,3,5-trién-7-amine

On ajoute 3,2 g du composé obtenu au stade 2 en solution dans 30 mL de THF à une suspension de 0,9 g de LiAlH₄ dans 20 mL de THF. On porte au reflux 1 heure 30 minutes puis on hydrolyse par 0,6 ml d'eau et 0,5 mL de soude à 20%, et enfin par 2,3 mL d'eau. Les sels minéraux sont ensuite filtrés, rincés au THF puis le filtrat obtenu est évaporé à sec. On obtient 2,3 g du composé attendu.

Rendement = 92%

### Exemple 4 : (7S)-3,4-diméthoxy-N-méthylbicyclo[4.2.0]octa-1,3,5-trién-7-amine D'après EP 0 534 859

On fait réagir l'amine obtenu à l'exemple 3 avec une quantité équimolaire de l'acide (d) camphosulfonique dans l'éthanol. Après évaporation sous vide du solvant, le sel est recristallisé une première fois dans l'acétate d'éthyle puis dans l'acétonitrile jusqu'à l'obtention de l'énantiomère cible avec une pureté optique supérieure à 99% (évaluation par HPLC sur colonne Chiralcel^{®} OD).

### Exemple 5 : 3-{3-[{[(7S)-3,4-diméthoxybicyclo[4.2.0]octa-1,3,5-trién-7-yl]méthyl}(méthyl)amino]propyl}-7,8-diméthoxy-1,3-dihydro-2H-3-benzazépin-2-one D'après EP 0 534 859

La solution de sel de (d) camphosulfonate obtenu à l'exemple 4 dans l'acétate d'éthyle est amenée à pH basique à l'aide d'hydroxyde de sodium puis la phase organique est séparée, lavée, séchée sue Na₂SO₄ et évaporée.

On porte ensuite au reflux pendant 18 heures un mélange composé de 5,6 g de carbonate de potassium, 2,2 g de l'amine ci-dessus dans 100 mL d'acétone et de 4g de 3-(3-iodopropyl)-7,8-diméthoxy-1,3-dihydro-2*H*-3-benzazépin-2-one.

On évapore le solvant sous vide, on reprend le résidu à l'acétate d'éthyle, puis on extrait à l'acide chlorhydrique 3N.

On amène la phase aqueuse décantée à pH basique à l'aide d'hydroxyde de sodium, puis on l'extrait à l'acétate d'éthyle. Après lavage à neutralité et séchage sur MgSO₄, on évapore sous vide pour obtenir 4,5 g d'une huile qui est purifiée sur colonne de silice en utilisant comme éluant un mélange dichlorométhane/méthanol (90/10).

Rendement = 64%

### Exemple 6 : 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

D'après EP 0 534 859 5 g du composé obtenu à l'exemple 5 dans 50 mL d'acide acétique glacial sont hydrogénés dans un appareil de Parr, sous un pression de 4,9 bar d'hydrogène à température ambiante pendant 24 heures, en présence d' 1 g d'hydroxyde de palladium à 10%. On filtre le catalyseur, on évapore le solvant, puis on reprend le résidu sec à l'eau et à l'acétate d'éthyle. On sèche la phase organique sur sulfate de magnésium anhydre, on concentre sous vide puis le résidu est purifié sur colonne de silice en utilisant comme éluant un mélange dichlorométhane/méthanol (95/5).

Après recristallisation dans l'acétate d'éthyle, on obtient 2g du composé attendu.

Rendement = 40%

P.F. = 101-103°C

## Revendications

1. Procédé de synthèse du composé de formule (I) : 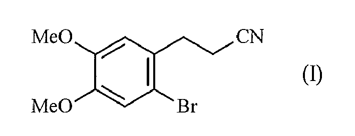 **caractérisé en ce que** le composé de formule (VIII) : 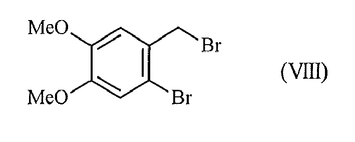 est soumis à l'action d'une base en présence d'acétonitrile dans un solvant organique pour conduire au composé de formule (I).

2. Procédé selon la revendication 1, **caractérisé en ce que** la base utilisée pour effectuer la transformation du composé de formule (VIII) en composé de formule (I) est choisie parmi le *n*-butyllithium, le diisopropylamidure de lithium, le bis(trimethylsilyl)amidure de potassium, le tert-butylate de potassium et l'hydroxyde de potassium.

3. Procédé selon la revendication 2, **caractérisé en ce que** la base utilisée pour effectuer la transformation du composé de formule (VIII) en composé de formule (I) est le *n-*butyllithium.

4. Procédé selon l'une quelconque des revendications 1 à 3, **caractérisé en ce que** le solvant organique utilisé pour effectuer la transformation du composé de formule (VIII) en composé de formule (I) est le tétrahydrofurane.

5. Procédé selon l'une quelconque des revendications 1 à 4, **caractérisé en ce que** la transformation du composé de formule (VIII) en composé de formule (I) est conduite à une température comprise entre -65°C et 25°C.

6. Procédé selon la revendication 1, **caractérisé en ce que** le composé de formule (VIII) est préparé à partir du composé de formule (IX) : 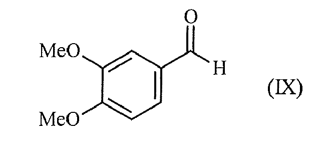 qui est transformé, par une réaction de réduction, en composé de formule (X) : 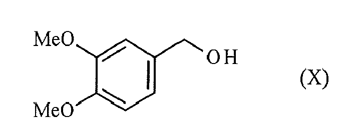 lequel est transformé, par une réaction de bromation, en composé de formule (VIII) :

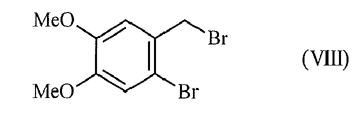

7. Procédé de synthèse de l'ivabradine, de ses sels pharmaceutiquement acceptables et de ses hydrates, **caractérisé en ce que** le composé de formule (VIII) est transformé en composé de formule (I) suivant le procédé de la revendication 1, puis le composé de formule (I) est transformé en ivabradine, qui peut être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.
